# EUROPEAN PATENT SPECIFICATION

(11) **EP 4 371 496 B1**
(45) Date of publication and mention of the grant of the patent: **12.08.2026**
(21) Application number: 23161129.4
(22) Date of filing: 10.03.2023
(51) Int. Cl.: A61B 6/03, A61B 6/00

(54) **COMPUTER-IMPLEMENTED METHOD FOR PREPARING A COMPUTED TOMOGRAPHY SCAN, COMPUTER PROGRAM AND COMPUTED TOMOGRAPHY SYSTEM**
COMPUTERIMPLEMENTIERTES VERFAHREN ZUR VORBEREITUNG EINES COMPUTERTOMOGRAFISCHEN SCANS, COMPUTERPROGRAMM UND COMPUTERTOMOGRAFIESYSTEM
PROCÉDÉ MIS EN OEUVRE PAR ORDINATEUR POUR PRÉPARER UN BALAYAGE DE TOMODENSITOMÉTRIE, PROGRAMME INFORMATIQUE ET SYSTÈME DE TOMODENSITOMÉTRIE

(43) Date of publication of application: 22.05.2024
(73) Proprietor: Siemens Healthineers AG, 91301 Forchheim (DE)
(72) Inventor: Allmendinger, Thomas, 91301 Forchheim (DE); Baer-Beck, Matthias, 91080 Spardorf (DE); Petersilka, Martin, 91325 Adelsdorf (DE); Prokein, Andreas, 91088 Bubenreuth (DE)
(74) Representative: Siemens Healthineers Patent Attorneys

(56) References cited:
- WO-A1-2013/049818
- US-A1- 2012 230 470
- US-A1- 2016 183 905

## Description

The invention relates to a computer-implemented method for preparing a computed tomography scan and a corresponding computer program, and computed tomography system.

Independent of the grammatical term usage, individuals with male, female or other gender identities are included within the term.

With the technical development in medical imaging also come higher demands are made regarding image quality. On the one hand, image quality, in particular in computed tomography (CT) imaging, can be influenced by the technical side, such as by the quality of the hardware. On the other hand, image quality can also be influenced by the choice of parameters that are used during the imaging process. Image quality may, for example, be characterized by a contrast-to-noise ratio. For example, in CT imaging, some scan parameters may be selected such that a contrast-to-noise ratio is optimized. A contrast-to-noise ratio usually is defined as a ratio of a difference between two signal intensities in a region of interest, in particular as numerator, and of a value describing image noise, in particular as denominator. The value describing the image noise may for example be a standard deviation of image noise. Image noise may be defined to be a random variation of the image signal, e.g., a random variation of a grey value, or of a colour value. Image noise may, for example, be created by electronic noise in the used hardware parts or in the power supply lines of the surrounding building or by mechanical vibrations.

In order to provide a good image quality for each respective demand, in the state of the art, a user may typically choose a scan protocol or a basic scan mode depending on what is to be examined and what kind of hardware is available. Hence, a user may choose a scan protocol that seems most suitable to produce good imaging data of an object or subject to be examined. The chosen scan protocol may then be further adapted to optimize the contrast-to-noise ratio.

However, in particular with advancing progress of technical possibilities, the demands of image quality are also increasing. Thus, it would be desirable to increase image quality even further. An option may be to provide a wider array of scan protocols and scan modes to the user, such that, having more choice, the user may find a more optimized solution for each individual case. However, having more options may also increase the complexity of imaging, and may thus lead to more problems and difficulties regarding maintenance and handling of a corresponding imaging system. Accordingly, the demands on the user's expertise may increase with the complexity of the imaging system, making it more difficult to run such an imaging system. Exemplary computer-implemented methods for preparing a computed tomography scan of a subject are disclosed in US 2016/183905 A1 and WO 2013/049818 A1.

It is therefore an object of the invention to provide a means to further improve image quality.

This object is met or exceeded by a method according to claim 1, a computer program according to claim 12, and a computed tomography system according to claim 13. Preferred embodiments are included as dependent claims

According to a first aspect of the invention, a computer-implemented method for preparing a computed tomography (CT) scan of a subject is provided. The method comprises the following steps:
(a) receiving scan-related information comprising subject-related information;
(b) automatically adapting at least two imaging parameters in order to optimize image quality by means of an optimization function, wherein the optimization function comprises the scan-related information as at least one constant and the imaging parameters as variables to be optimized, wherein optimizing image quality comprises reducing scan artifacts of the computed tomography scan;
(c) providing the adapted imaging parameters , wherein the optimization function comprises a linear combination of a plurality of sub-cost functions, each sub-cost function representing another aspect of image quality, wherein one of the sub-cost functions is a cost function designed to reduce scan artifacts and another one of the sub-cost functions is a cost function that is designed to increase the contrast-to-noise ratio, and in that the imaging parameters comprise at least one reconstruction parameter, the at least one reconstruction parameter being a keV level of reconstruction, wherein the keV level of reconstruction has an impact on both the scan artifacts and the contrast-to-noise ratio.

The subject may in particular be a human being or animal, in particular a patient. Scan-related information may preferably be defined as information that is relevant for the CT scan, in particular for the image quality. The scan-related information comprises subject-related information, i.e., information about the subject that is related to the scan. Hence, subject-related information may preferably be defined to be information about the subject that is relevant for the CT scan, in particular for the image quality. Subject-related information may be patient-related information, e.g., when the subject is a patient. The scan-related information may, for example, comprise a contrast-related information, such as body tissue characteristics and/or contrast medium concentration. Subject-related information may, for example, comprise geometric information of the subject and/or an age or weight of the subject. Geometric information may, for example, concern a size or diameter of the subject.

Imaging parameters may preferably comprise scan parameters and reconstruction parameters. In some cases, it may be feasible to only take into account either scan parameters or reconstruction parameters. However, in most cases, taking into account both will be advantageous, e.g., to achieve a more well-rounded optimization of image quality. Scan parameters are in particular parameters that are applied during a CT scan, such as tube voltage, filter setting, tube current, and detector collimation, to acquire raw data. For example, tube voltage may determine the photon energy of the X-ray beam and thus influence the contrast but also the noise. Usually, a CT system provides the possibility to set different tube voltages for a CT scan. Increasing tube current may reduce noise but may also increase a radiation dose. Reconstruction parameters, such as setting the keV level of monoenergetic image reconstructions in spectral scan modes, are in particular parameters that are applied to reconstruct image data from the acquired raw data. Setting keV levels may, for example, be used to enhance the contrast between different materials. In particular, during reconstruction, several slices of the subject may be calculated which, put together, may result in three-dimensional image data of the subject.

The optimization function comprises the scan-related information and the imaging parameters. Hence optimizing the optimization function may be done with regard to the scan-related information and the imaging parameters. Since the scan-related information is provided as a constant and the imaging parameters are a variable, optimizing the optimization function may in particular comprise adjusting the imaging parameters, preferably such that the optimization function is minimized or that the optimization function is maximized. The optimization function is a cost function. For example, assuming scan and reconstruction parameters {p_{scan};p_{recon}} as imaging parameters, the adapted imaging parameters may comprise optimized scan and reconstruction parameters $\left\{{p}_{scan}^{opt}; {p}_{recon}^{opt}\right\}$. Preferably, *p*_{scan} and *p*_{recon} may both be vectors, each containing a collection of one or several scan parameters and at least one reconstruction parameter, respectively. Hence, exemplarily, the adapted imaging parameters may be determined by minimizing a cost function in the form of $\left\{{p}_{scan}^{opt}; {p}_{recon}^{opt}\right\} = argmin \left\{Cost\left({p}_{scan}, {p}_{recon}, c\right)\right\} ,$ where *c* is a constant describing the scan-related information regarding the subject. Therein, "Cost" is a cost function that is to be optimized by minimizing it via adjusting the imaging parameters {p_{scan};p_{recon}} while *c* is kept constant. The imaging parameters comprise a keV level for reconstruction [keV], and may also be a wedge filter setting [WF], a tube voltage [*U*] and a tube current [mAs], such that the function for seeking to optimize the cost function may take the form $\left\{{keV}^{opt}{, WF}^{opt},{U}^{opt},{mAs}^{opt}\right\} = argmin \left\{Cost\left(keV, WF, U, mAs, c\right)\right\} .$

In an embodiment not forming part of the present invention the optimization function may be designed to only optimize scan quality via reducing scan artifacts. For example, the cost function *Cost* may be a cost function *S*_{Artifact} designed to reduce artifacts. For example, the cost function *S*_{Artifact} may be generated empirically by running phantom scans and assessing the image quality of thereby generated images by using physical models of the imaging and reconstruction process or by a combination of both. The optimization function is designed to optimize multiple aspects of scan quality, namely reducing scan artifacts and increasing a contrast-to-noise ratio. It may be an option to decouple the optimization of different aspects of image quality, in particular of reducing scan artifacts and of another aspect of image quality, such as contrast-to-noise ratio. Hence, there may be two steps of optimization, wherein first, a first aspect of image quality is optimized, thereby adapting at least one first imaging parameter, and then, a second aspect of image quality is optimized, thereby adapting at least one second imaging parameter, while the at least one first imaging parameter is kept fixed. In particular, the first aspect is a contrast-to-noise ratio, and the second aspect is reducing scan artifacts, such as scatter artifacts. The at least one first imaging parameter may for example be one or several of the following: a tube current profile, a tube voltage, a keV level. The at least one second imaging parameter may for example be a wedge filter setting.

The optimization function is preferably designed such that its optimization leads to an improved image quality. Image quality may be a measure for reliability of the acquired image data and/or information derivable from the acquired image data. Image quality may be characterized by different aspects. Aspects of image quality may, for example, comprise image contrast, image noise and spatial resolution. Usually, a higher image contrast as well as lower noise level is desirable. Thus, depending on the clinical task as well as the boundary conditions thus as an applied contrast agent, a measure of image quality that may be used is the contrast-to-noise ratio. According to the invention, an aspect of image quality are in particular scan artifacts. Scan artifacts may, for example, be caused by scattering, such as forward scattering or cross scattering. Cross scattering may in particular occur in Dual Source CT. Further causes of scan artifacts may be photon starvation, beam hardening, e.g., due to metals, cone beam with respect to detector collimation, movement of the subject, and/or spectral separation. The inventors have found that with increasing technical standards of CT imaging, scan artifacts can have a larger impact on image quality, in particular in terms of reliability, then previously thought. Usually, according to the state of the art, there is not provided an automatic adaption of imaging parameters based on (potential) scan artifacts. Here, it has been found that, especially in non-standard circumstances such as particularly large subjects, scan artifacts can play a significantly important role for image quality that should advantageously not be neglected. Taking into account scan artifacts may lead to a more complex calculation of imaging parameters. Furthermore, an optimization of the imaging parameters with regard to scan artifacts may, for example, potentially lead to a less optimized contrast-to-noise ratio because the imaging parameters are determined based on additional restraints, namely to reduce scan artifacts. Nevertheless, it has been found that, depending on the subject to be examined, the optimization with regard to scan artifacts may still lead to a better overall image quality, in particular with respect to reliability and/or information contained in the image data. Thus, it is advantageous to optimize scan quality (at least partly) based on seeking to reduce scan artifacts in order to take into account effects of image artifacts.

The method may, for example, be carried out by a processing unit such as a CPU, a GPU, a computer or part of a computer. The computer may be a PC, a server, and/or a control unit of a computed tomography (CT) system. The computer may also be a mobile device, such as a laptop, tablet computer or mobile phone. The control unit may in particular comprise a dedicated workflow engine, e.g., comprising a computer program configured to carry out the corresponding method steps. The adapted imaging parameters may in particular be provided to the CT system before the start of a CT scan.

The optimization function comprises a linear combination of a plurality of sub-cost functions, each sub-cost function representing another aspect of image quality. In particular, each sub-cost function may be designed such that optimizing it leads to reducing the corresponding aspect of image quality. In particular, the optimization function may be a cost function comprising the linear combination of a plurality of sub-cost functions. Accordingly, seeking to optimize the optimization function may lead to reducing the overall image quality taking into account the different aspects of image quality. An aspect of image quality may be defined such that it is a criterion that influences image quality. One aspect of image quality is in particular an occurrence of image artifacts. Further aspects of image quality may, for example comprise one or several of the following: an image contrast, image noise, in particular a contrast-to-noise ratio, and a spatial resolution. The sub-cost functions may each have a pre-factor. The pre-factors may be designed to normalize the different sub-cost functions and/or to weight the different sub-cost functions with respect to each other. For example, the sub-cost functions may be weighted with respect to their impact on the overall image quality. Considering several sub-cost functions cost*ᵢ*, the above-mentioned cost function *Cost* may thus, for example, take the form $Cost \left({p}_{scan}, {p}_{recon}, c\right) = {\sum }_{i} {λ}_{i} ⋅ {cost}_{i} \left({p}_{scan}, {p}_{recon}, c\right) ,$ where *λᵢ* are the pre-factors of the different sub-cost functions cost*ᵢ*. Therein, each sub-cost functions costᵢ describes an aspect of image quality that is to be optimized. One of the sub-cost functions cost*ᵢ* is a cost function designed to reduce scan artifacts *S*_{Artifact}. Another sub-cost function is a cost function *C*_{CTNR} that is designed to increase the contrast-to-noise ratio.

According to an embodiment, the optimization function comprises at least one penalty term, preferably a plurality of penalty terms. According to an embodiment, at least one of the plurality of sub-cost functions is or comprises a penalty term, the penalty term in particular being weighted by a weighting parameter. The weighting parameter may correspond to the pre-factor mentioned above. It may be provided that several of the sub-cost functions are or comprise a penalty term. The at least one penalty term or, respectively, each penalty term may comprise at least one of the imaging parameters as a penalty parameter. The weighting parameter may be chosen such that it corresponds to a measure of the impact of the corresponding at least one imaging parameter on the overall image quality. Advantageously, the penalty term or preferably a plurality of penalty terms may be an efficient way of considering different aspects of image quality. According to an embodiment, at least one penalty term relates at least one of the scan-related information to at least one of the imaging parameters. For example, a patient size may be related to a wedge filter setting or to a CT scan mode to be chosen. Hence, advantageously, the corresponding imaging parameter may be optimized with regard to the at least one scan-related information.

According to an embodiment, the optimization function comprises a dose response function, the dose response function depending on the at least two imaging parameters. The dose response function may correspond to a radiation dose that is applied to the subject during a CT scan. The dose response function may be designed such, that a Computed Tomography Dose Index (CTDI) is minimized during optimization. The CTDI is a standard measure of dosimetry and suitable as basis for the calculation of the radiation exposure of the subject during the CT scan. Due to detrimental effects on health of the subject caused by radiation, it is usually a goal to minimize the radiation dose the subject is exposed to as much as possible. On the other hand, an increased radiation dose may be necessary to improve image quality, e.g., by increasing the tube current. Accordingly, providing the dose response function as part of the optimization function may advantageously allow to take this aspect into account when adapting the imaging parameters. The dose response function may be of the form $D \left({p}_{scan}, {p}_{recon}, c\right) .$

In particular the dose response function may be part of the linear combination of sub-cost functions, representing an additional cost function. In an embodiment not forming part of the present invention, the dose response function may, optionally, not be designed to improve image quality but to reduce radiation dose. However, alternatively, the dose response function may preferably be designed to take into account both, a reduction of a radiation dose and an optimization of contrast-to-noise ratio. For example, the cost function may thus be in the form of $Cost \left({p}_{scan}, {p}_{recon}, c\right) = α D \left({p}_{scan}, {p}_{recon}, c\right) + {\sum }_{i} {λ}_{i} ⋅ {cost}_{i} \left({p}_{scan}, {p}_{recon}, c\right) ,$ where α is the pre-factor of the dose response function and cost*ᵢ* are the remaining sub-cost functions with pre-factors *λᵢ*. Preferably the dose response function depends on all imaging parameters that are considered in the entire cost function, i.e., in all sub-cost functions.

According to an embodiment, the scan-related information comprises a size of the subject. In particular, if the subject is a patient, the size of the subject may be a patient size. The patient size may preferably be determined automatically. For example, the subject size may be derived from a topogram that is taken at the beginning of the CT examination in which the CT scan whose imaging parameters are adapted is to be acquired. A topogram is typically a 2-dimensional X-ray image acquired using a CT scanner. The topogram is routinely used in clinical CT scanning to define the scan range of the subsequent CT scan. Alternatively, the subject size may be determined from a camera image, in particular a camera image taken at the beginning of the CT examination in which the CT scan, whose imaging parameters are adapted, is to be acquired. E.g., the CT system may comprise a camera and be configured to automatically take a camera image of the subject and determine the size of the subject from the camera image. The camera may be a 2D camera or a 3D camera. It may be installed above the patient bed, for example fixed at the ceiling. Alternatively, the patient size may be input by a user or retrieved from a database. According to an embodiment, the scan-related information comprises a diameter of the subject at a region of interest that is to be scanned. Hence, the subject size may be defined by the diameter of the subject. It has been found that large patients may lead to particularly strong artifacts at least in some kind of CT imaging examinations, e.g., during cardiac CT imaging with photon counting detectors. This can be explained by a combination of effects. First, primary intensities of the CT imaging signal may be particularly low for large subjects due to damping of the signal in the subject, e.g., in a patient's body. Further, also a scatter intensity typically increases with subject size, since a larger subject diameter leads to a longer way of the x-ray beam through the subject and thus to more opportunities for scattering. Both these factors are typically exponentially dependent on the subject's diameter. Hence, particularly large subjects can lead to strong artifacts, whereas basic models assuming an averaged size subject may not show any significant impact of scan artifacts. Hence considering both the subject size and its impact on scan artifacts may advantageously lead to an improved image quality, to an even greater effect than more basic models might indicate.

According to an embodiment, the imaging parameters comprise a wedge filter setting. The wedge filter setting may, for example, concern the geometric properties of a wedge filter. The wedge filter setting may concern a width of the wedge filter. Usually, according to the state of the art, a wedge filer, e.g., a cardio wedge, is designed that a resulting dose is chosen with regard to the majority of subjects. However, in particular for very large subjects or subjects with unusual anatomy, it has been found that such a standard wedge may be non-optimal for image quality in some cases. For example, while large subjects or unusual anatomy, such as implants, may lead to reduced imaging signal intensities, these already reduced intensities are further reduced by the wedge filter. Additionally, also the scatter intensity increases with the subject's size. The decreased signal intensity, due to subject size and wedge filter combined, may now be so low that a decrease in scatter intensity due to the wedge filter may no longer be enough to result in a net positive effect of the wedge filter with regard to the image quality. For example, a large subject size may lead to a significant increase in scattering being directed essentially parallel to the x-ray beam, e.g., forward scattering that is not filtered as efficiently as cross scattering by the wedge filter. Hence, in this case it may be beneficial for the image quality to use another wedge filter, e.g., with a wider width, to increase the imaging signal intensity.

According to an embodiment, adapting imaging parameters comprises, depending on the scan-related information, in particular depending on the subject size, determining a wedge filter to be used out of a plurality of at least two available wedge filters. The plurality of wedge filters may preferably differ from each other. In particular the geometric properties of the applied wedge filter may be set by choosing from the plurality of available wedge filters, wherein the available wedge filters differ from each other with respect to their geometric properties. Additionally and/or alternatively the wedge filters may differ with respect to their material properties. For example, a typical wedge filter used for cardio imaging, i.e., a cardio wedge, may have a shape that is designed such that a dose distribution is focused to an inner part of the scan field of view of the scanner, namely the heart region. This can be achieved by providing an increasing thickness of the wedge with increasing distance from the isocenter. This may allow, that regions are located in the periphery of the x-ray fan, e.g., the lungs, receive less radiation exposure. While such a standard geometry may be suitable for the majority of subjects, the geometry may be less optimal for unusual subjects, e.g., particularly large subjects. Namely, the increasing thickness of the wedge that reduces the dose towards an outer region may also increase the ratio of scatter to signal intensity of photons reaching the CT detector. This may lead to a higher scatter artifact level in the reconstructed image. Accordingly, it may be advantageous to replace the standard cardio wedge with another wedge filter more appropriate for this particular subject. For example, a wedge filter that has a muss less pronouncedly shaped profile may be more beneficial for large subjects. This may lead to the signal intensities in the periphery being increase and therefore the scatter to signal intensity ratio and, consequently, the artifacts in this region may be reduced as well.

According to an embodiment, adapting imaging parameters comprises choosing a computed tomography (CT) scan mode out of a plurality of available computed tomography scan modes. A CT scan mode to be chosen from may for example be a dual source mode versus a single source mode or a multi energy mode versus a single energy mode. Further CT scan modes may be spectral scan modes, such as TwinBeam, TwinSpiral, or Photon Counting modes. For example, the CT scan mode may be optimized to achieve a desired spectral separation needed for a specific task, such as monoenergetic, VNC, bone marrow. Preferably the optimization may be based on a subject size, in particular a subject diameter. For example, while dual source CT has some particular, well-known benefits versus single source CT, the overall scatter intensity tends to be higher due to cross scattering from the first source to the second detector and from the second source to the first detector. This may impact a negative effect of a standard cardio wedge filter as described above even more.

According to an embodiment, the imaging parameters are scan parameters comprising at least one of the following: a kV filter setting, a spectral filter setting, a collimation setting, a spiral pitch factor and a rotation time setting. Higher kV filter settings may enable to reduce beam hardening or photon starvation artifacts. Higher kV levels may be particularly beneficial for large subject diameters. A removal of spectral filters may be beneficial for large subject diameters. The detector collimation may in particular influence a spatial resolution. A smaller collimation size usually leads to a higher spatial resolution and may reduce artifacts, such as scatter artifacts. However, a smaller detector collimation may also lead to an increased noise. Hence, there are conflicting effects of the collimation size. It has been found that these effects may depend heavily on the subject size.

The spiral pitch factor may determine the scan speed and a speed of a patient table moving through the CT scanner. The spiral pitch factor and/or the rotation time setting may be adapted depending on the subject size. This may be beneficial in order to reduce scan artifacts.

According to the invention, the imaging parameters comprise at least one reconstruction parameter, the at least one reconstruction parameter being a keV level of reconstruction. The keV level of reconstruction may have an impact on both scan artifacts and a contrast-to-noise ratio. For example, photon counting CT detectors, may enable to apply dual energy image reconstruction methods, allowing the generation of monochromatic images, in particular also in the field of cardiac CT scanning. The optimal keV level at which monochromatic images of angiographies are displayed is typically in the low energy regime, e.g., between 50 keV and 60 keV. Relatively to the primary x-rays, namely the x-rays contributing to the signal intensity, scattered x-rays typically have a lower energy level since they have lost parts of their energy during the scattering. If images are reconstructed at lower levels of keV, this may increase the scatter to primary x-ray ratio and thereby the scatter artifact level in the reconstructed images. Since the keV level of the reconstruction thus may impact the final image quality with respect to scatter artifacts it may be desirable to include this reconstruction parameter in the optimization calculation.

The embodiments described herein may be combined with each other unless indicated otherwise. For example, combining several of the above embodiments, a cost function or sub-cost function designed to reduce scan artifacts *S*_{Artifact}, may regard a patient size, in particular patient diameter *d*_{Patient} and a wedge filter setting *WF*. A further sub-cost function *C*_{CTNR} may be designed to increase the contrast-to-noise ratio. An overall optimization function for a combined optimization of a wedge filter setting, the keV level of reconstruction, and a contrast-to-noise ratio on a photon counting CT system may take the form $\begin{matrix}Cost \left(keV,WF,U,mAs c\right) \\ = {λ}_{1} ⋅ {S}_{Artifact} \left(keV, {d}_{Patient}, WF\right) + {λ}_{2} ⋅ {C}_{CTNR} \left(MC, U, {d}_{Patient}, keV, mAs\right)\end{matrix}$ with the pre-factors *λ*₁ and *λ*₂ that are used to weight the impact of the two cost functions. Via the pre-factors the tradeoff between the optimization with regard to artifacts and the optimization with regard to contrast-to-noise ratio may be influenced and controlled. MC is a material contrast, U is a tube voltage, and mAs is a tube current. Therein, [keV] may be a reconstruction parameter, [WF, U, mAs] may be scan parameters and [MC, *d*_{Patient}] may be scan-related information that are kept constant when calculating the optimization. Advantageously, in this particular example, applying the linear combination allows to optimize the keV level because the keV level has an impact on both the artifacts and the contrast-to-noise ratio. A low keV level may lead to higher scatter artifacts and, at the same time to a better iodine-water contrast-to-noise ratio. Similarly, applying a linear combination may be beneficial in other cases, where an imaging parameter has an effect on different aspects of image quality. In an alternative example not forming part of the present invention, the optimization of the image quality may comprise reducing the scan artifacts separately. This may for example be an option, when the keV level of reconstruction is determined with respect to the contrast-to-noise ratio and is thus not used as a variable in order to reduce scan artifacts. In this case, the cost function may, for example, be of the form $Cost \left(WF, c\right) = {S}_{Artifact} \left({d}_{Patient}, WF\right)$

According to a further aspect of the invention, a computer program is provided that comprises instructions which, when the program is executed by a computer, cause the computer to carry out the method as described herein. All features and advantages of the method may be adapted to the computer program and vice versa.

A computer-readable storage medium, in particular non-transient storage medium, may be provided that comprises instructions which, when executed by a computer, cause the computer to carry out the method as described herein. All features and advantages of the method and of the computer program may be adapted to the computer-readable storage medium and vice versa.

According to a further aspect of the invention, a computed tomography (CT) system comprising a processing unit that is configured to carry out the method as described herein is provided. All features and advantages of the method, of the computer program and of the computer-readable storage medium may be adapted to the computed tomography system and vice versa.

The accompanying drawings illustrate various exemplary embodiments and methods of various aspects of the invention.
Fig. 1 shows a flow diagram of a method according to an embodiment of the invention;
Fig. 2 shows examples of relations between scan-related information and imaging parameters of four penalty terms;
Fig. 3 depicts the problem of cross scattering in dual source mode;
Fig. 4 shows a computed tomography system 1 according to an embodiment of the invention.

Similar elements are designated with the same reference signs in the drawings.

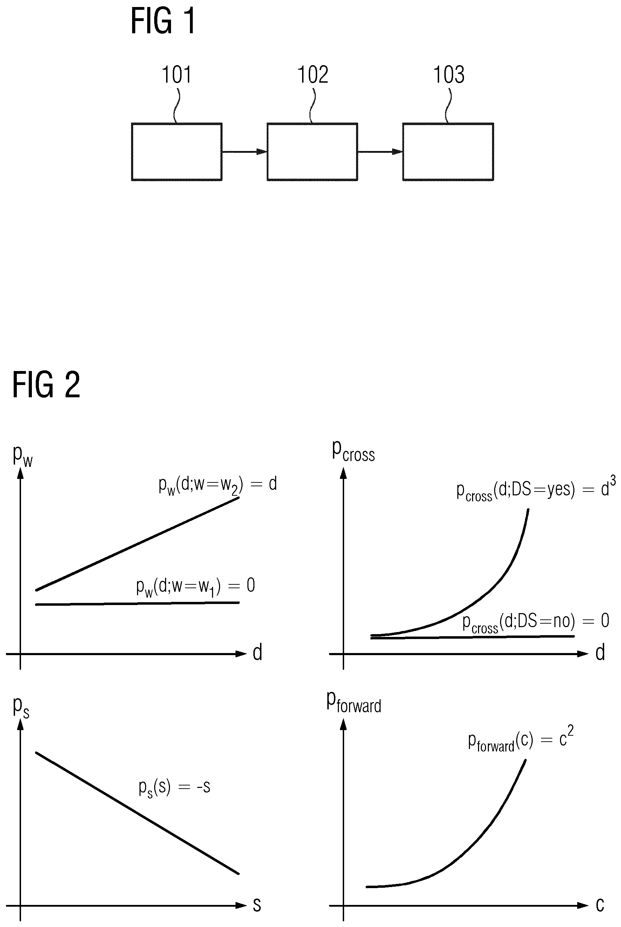

Figure 1 shows a flow diagram of a method according to an embodiment of the invention. The method is designed for preparing CT scan of a subject 5. Therein, in a first step 101, scan-related information comprising subject-related information is received by a processing unit 2 of a CT system 1. The subject-related information may in particular comprise a size of the subject 5, e.g., defined by a diameter of the subject 5. In a further step 102, at least two imaging parameters are adapted based on seeking to optimize image quality via an optimization function. The imaging parameters may for example be a wedge filter setting, a kV filter setting, a spectral filter setting, a collimation setting, a spiral pitch factor, or a rotation time setting. The imaging parameters comprise at least one reconstruction parameter, the at least one reconstruction parameter being a keV level of reconstruction. The optimization function comprises the scan-related information as at least one constant and the imaging parameters as variables to be optimized. In the optimization step, optimizing the image quality comprise reducing scan artifacts of the computed tomography scan. In a further step 103 the adapted imaging parameters are provided, e.g., to be used during a following CT scan and image reconstruction.

The optimization function comprises a linear combination of a plurality of sub-cost functions each sub-cost function representing another aspect of scan image quality. The sub-cost functions are in the form of penalty terms that are weighted by pre-factors that serve as weighting parameters. The optimization function further comprises a dose response function *D* that depends on the imaging parameters and that is also part of the linear combination in this example. Preferably optimizing may be achieved by minimizing the linear combination, which may for example have the following form: $argmin \left[αD\left(kV; c; {w}_{x}\right)+{βp}_{w}\left(d; {w}_{x}\right)+{γp}_{s}\left(s\right)+{δp}_{cross}\left(d, DS\right)+{εp}_{forward}\left(c\right)\right]$

Hence, there is a linear combination of the dose response function *D* and several penalty terms *p*. The dose response functions and the penalty terms are weighted by pre-factors (*α, β, γ, δ, ε*). The penalty terms are depicted as functions in figure 2. A first penalty term, *p_{w},* describes two different variants of wedge filters having different widths, namely *w*₁ and *w*₂. The wedge filters may differ in further characteristics, e.g., their overall geometric shape. The wedge filters are plotted against a subject diameter, *d*. As can be seen, the course of the graph is different for both wedge filters. This difference, i.e., the respective (linear) dependency with regard to the subject diameter is put into the penalty term. This penalty term or another penalty term (not included in this example) may further comprise information about a contrast-to-noise ratio influenced by the wedge setting. Another penalty term, *p*_{cross}, describes a dependance of cross scattering on the patient diameter, *d*, both for dual source (DS=yes) and for single source (DS=no). Accordingly, depending on patient size single source or dual source may be more beneficial for image quality. Another penalty term, *pₛ*, generally defines a faster acquisition speed, *s*, as favorable, thus increasing the penalty for lower speed (in this case, the relation is inversely linear). This penalty term may be used to aim for a faster acquisition speed, as far as this is feasible, e.g., to avoid getting to slow acquisition speeds via this method. Another penalty term, *p_{forward},* describes the dependency of forward scattering on a collimator width, *c*, which increases exponentially to the power of 2 in this example. The given penalty terms are to be understood as examples. Further penalty terms may be added to further refine the optimization.

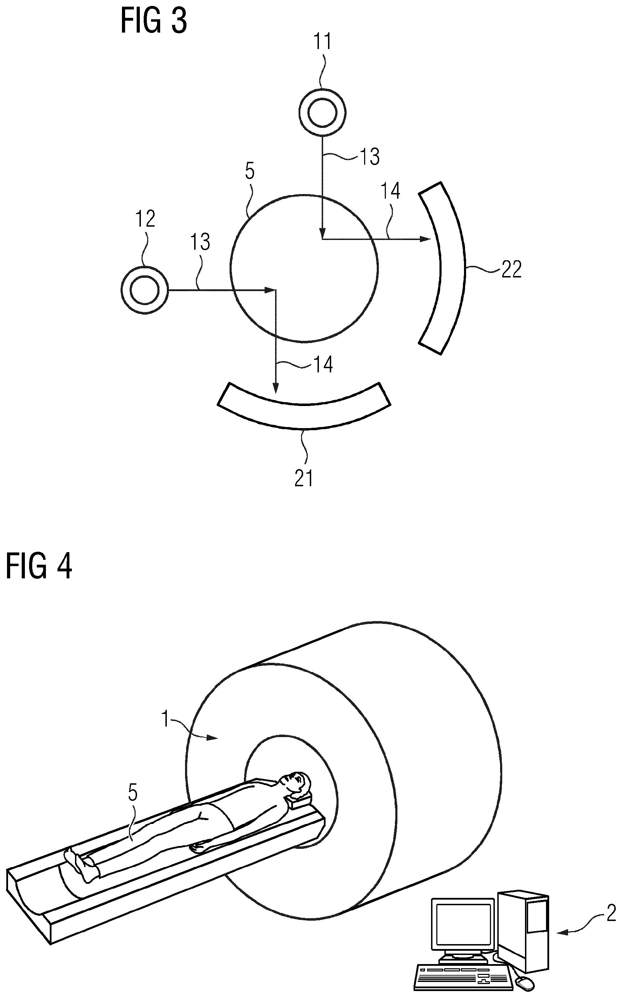

Figure 3 depicts the problem of cross scattering in dual source mode. In dual source mode, two x-ray sources, a first x-ray source 11 and a second x-ray source 12 are used together with a corresponding first detector 21 and second detector 22. Primary x-rays 13 from the first source 11 are detected by the first detector 21 and primary x-rays 13 from the second source 12 are detected by the second detector 22. However, due to scattering in the object 5, there are also scattered x-rays 14. Due to this cross scattering some scattered x-rays 14 from the first source 11 also reach the second detector 22 and some scattered x-rays 14 from the second source 12 also reach the first detector 21, leading to cross-scatter artifacts. This effect can be significantly stronger for a subject 5 with a large diameter, since the longer way of the primary x-rays through the subject 5 leads to more opportunities for scattering and thus to the occurrence of more cross scattering.

Figure 4 shows a computed tomography (CT) system 1 according to an embodiment of the invention. The CT system 1 comprises a processing unit 2 that is configured to carry out the method as described herein. The processing unit 2 in this example is depicted as a personal computer. The processing unit 2 may in particular be a control unit of the CT system 1.

## Claims

1. A computer-implemented method for preparing a computed tomography scan of a subject (5), the method comprising the following steps:
(a) receiving scan-related information comprising subject-related information;
(b) automatically adapting at least two imaging parameters by means of an optimization function in order to optimize image quality,
wherein the optimization function comprises the scan-related information as at least one constant and the imaging parameters as variables to be optimized,
wherein optimizing image quality comprises reducing scan artifacts of the computed tomography scan;
(c) providing the adapted imaging parameters;
**characterised in that** the optimization function comprises a linear combination of a plurality of sub-cost functions, each sub-cost function representing another aspect of image quality, wherein one of the sub-cost functions is a cost function designed to reduce scan artifacts and another one of the sub-cost functions is a cost function that is designed to increase the contrast-to-noise ratio, and **in that** the imaging parameters comprise at least one reconstruction parameter, the at least one reconstruction parameter being a keV level of reconstruction, wherein the keV level of reconstruction has an impact on both the scan artifacts and the contrast-to-noise ratio.

2. The computer-implemented method according to claim 1, wherein at least one of the plurality of sub-cost functions is a penalty term, the penalty term in particular being weighted by a weighting parameter.

3. The computer-implemented method according to claim 2, wherein at least one penalty term relates at least one of the scan-related information to at least one of the imaging parameters.

4. The computer-implemented method according to any one of the preceding claims,
wherein the optimization function comprises a dose response function, the dose response function depending on the at least two imaging parameters.

5. The computer-implemented method according to any one of the preceding claims,
wherein the scan-related information comprises a size of the subject (5), in particular a patient size.

6. The computer-implemented method according to any one of the preceding claims,
wherein the scan-related information comprises a diameter of the subject (5) at a region of interest that is to be scanned.

7. The computer-implemented method according to any one of the preceding claims 5 or 6,
comprising the steps of
- receiving a topogram or a camera image of the subject, wherein the topogram or camera image has preferably been acquired at the beginning of the CT examination in which the CT scan whose imaging parameters are adapted is to be acquired;
- deriving the subject size or diameter from the topogram or camera image.

8. The computer-implemented method according to any one of the preceding claims,
wherein the imaging parameters comprise a wedge filter setting.

9. The computer-implemented method according to any one of the preceding claims,
wherein adapting imaging parameters comprises, depending on the scan-related information, in particular depending on the subject size, determining a wedge filter to be used out of a plurality of at least two available wedge filters.

10. The computer-implemented method according to any one of the preceding claims,
wherein adapting imaging parameters comprises choosing a computed tomography scan mode out of a plurality of available computed tomography scan modes.

11. The computer-implemented method according to any one of the preceding claims,
wherein the imaging parameters are scan parameters comprising at least one of the following: a kV filter setting, a spectral filter setting, a collimation setting, a spiral pitch factor and a rotation time setting.

12. A computer program comprising instructions which, when the program is executed by a computer, cause the computer to carry out the method of any one of the preceding claims.

13. A computed tomography system (1) comprising a processing unit (2) that is configured to carry out the method according to any one of claims 1 to 11.

## Patentansprüche

1. Computerimplementiertes Verfahren zum Vorbereiten eines computertomografischen Scans eines Subjekts (5), wobei das Verfahren die folgenden Schritte umfasst:
(a) Empfangen von scanbezogenen Informationen, umfassend subjektbezogene Informationen;
(b) automatisches Anpassen von mindestens zwei Bildgebungsparametern mittels einer Optimierungsfunktion, um die Bildqualität zu optimieren,
wobei die Optimierungsfunktion die scanbezogenen Informationen als mindestens eine Konstante und die Bildgebungsparameter als zu optimierende Variablen umfasst,
wobei das Optimieren der Bildqualität das Reduzieren von Scanartefakten des computertomografischen Scans umfasst;
(c) Bereitstellen der angepassten Bildgebungsparameter;
**dadurch gekennzeichnet, dass** die Optimierungsfunktion eine lineare Kombination mehrerer Unterkostenfunktionen umfasst, wobei jede Unterkostenfunktion einen anderen Aspekt der Bildqualität repräsentiert, wobei eine der Unterkostenfunktionen eine Kostenfunktion ist, die dazu ausgelegt ist, Scanartefakte zu reduzieren, und eine andere der Unterkostenfunktionen eine Kostenfunktion ist, die dazu ausgelegt ist, das Kontrast-Rausch-Verhältnis zu erhöhen, und dadurch, dass die Bildgebungsparameter mindestens einen Rekonstruktionsparameter umfassen, wobei der mindestens eine Rekonstruktionsparameter ein keV-Rekonstruktionspegel ist, wobei der keV-Rekonstruktionspegel eine Auswirkung sowohl auf die Scanartefakte als auch auf das Kontrast-Rausch-Verhältnis hat.

2. Computerimplementiertes Verfahren nach Anspruch 1,
wobei mindestens eine der mehreren Unterkostenfunktionen ein Bestrafungsterm ist, wobei der Bestrafungsterm insbesondere durch einen Gewichtungsparameter gewichtet wird.

3. Computerimplementiertes Verfahren nach Anspruch 2,
wobei mindestens ein Bestrafungsterm mindestens eine der scanbezogenen Informationen mit mindestens einem der Bildgebungsparameter in Beziehung setzt.

4. Computerimplementiertes Verfahren nach einem der vorhergehenden Ansprüche,
wobei die Optimierungsfunktion eine Dosis-Wirkungs-Funktion umfasst, wobei die Dosis-Wirkungs-Funktion von den mindestens zwei Bildgebungsparametern abhängt.

5. Computerimplementiertes Verfahren nach einem der vorhergehenden Ansprüche,
wobei die scanbezogenen Informationen eine Größe des Subjekts (5), insbesondere eine Patientengröße, umfassen.

6. Computerimplementiertes Verfahren nach einem der vorhergehenden Ansprüche,
wobei die scanbezogenen Informationen einen Durchmesser des Subjekts (5) bei einer interessierenden Region umfassen, die gescannt werden soll.

7. Computerimplementiertes Verfahren nach einem der vorhergehenden Ansprüche 5 oder 6,
umfassend die folgenden Schritte:
- Empfangen eines Topogramms oder eines Kamerabildes des Subjekts, wobei das Topogramm oder Kamerabild vorzugsweise zu Beginn der CT-Untersuchung erfasst wurde, bei der der CT-Scan, dessen Bildgebungsparameter angepasst werden, erfasst werden soll;
- Ableiten der Subjektgröße oder des Durchmessers aus dem Topogramm oder dem Kamerabild.

8. Computerimplementiertes Verfahren nach einem der vorhergehenden Ansprüche,
wobei die Bildgebungsparameter eine Keilfiltereinstellung umfassen.

9. Computerimplementiertes Verfahren nach einem der vorhergehenden Ansprüche,
wobei das Anpassen von Bildgebungsparametern in Abhängigkeit von den scanbezogenen Informationen, insbesondere in Abhängigkeit von der Subjektgröße, Bestimmen eines zu verwendenden Keilfilters aus mehreren von mindestens zwei verfügbaren Keilfiltern, umfasst.

10. Computerimplementiertes Verfahren nach einem der vorhergehenden Ansprüche,
wobei das Anpassen von Bildgebungsparametern Auswählen eines Computertomografie-Scanmodus aus mehreren verfügbaren Computertomografie-Scanmodi umfasst.

11. Computerimplementiertes Verfahren nach einem der vorhergehenden Ansprüche,
wobei die Bildgebungsparameter Scanparameter sind, umfassend mindestens eines aus Folgendem: eine kV-Filtereinstellung, eine Spektralfiltereinstellung, eine Kollimationseinstellung, einen Spiralsteigungsfaktor und eine Rotationszeiteinstellung.

12. Computerprogramm, umfassend Anweisungen, die, wenn das Programm durch einen Computer ausgeführt wird, den Computer veranlassen, das Verfahren nach einem der vorhergehenden Ansprüche auszuführen.

13. Computertomografiesystem (1), umfassend eine Verarbeitungseinheit (2), die dazu ausgelegt sind, das Verfahren nach einem der Ansprüche 1 bis 11 auszuführen.

## Revendications

1. Un procédé mis en œuvre par ordinateur de préparation d'un balayage informatisé de tomodensitométrie d'un sujet (5), le procédé comprenant les stades suivants :
(a) recevoir une information se rapportant au balayage comprenant une information se rapportant au sujet ;
(b) adapter automatiquement au moins deux paramètres d'imagerie au moyen d'une fonction d'optimisation afin d'optimiser la qualité de l'image,
dans lequel la fonction d'optimisation comprend l'information se rapportant au balayage comme au moins une constante et des paramètres d'imagerie comme variables à optimiser,
dans lequel optimiser la qualité de l'image comprend réduire des artefacts du balayage informatisé de tomodensitométrie ;
(c) donner des paramètres d'imagerie adaptés ;
**caractérisé en ce que** la fonction d'optimisation comprend une combinaison linéaire d'une pluralité de fonctions de sous-coût, chaque fonction de sous-coût représentant un autre aspect de la qualité de l'image, dans lequel l'une des fonctions de sous-coût est une fonction de coût conçue pour réduire des artefacts de balayage et une autre des fonctions de sous-coût est une fonction de coût, qui est conçue pour augmenter le rapport contraste à bruit, et **en ce que** les paramètres d'imagerie comprennent au moins un paramètre de reconstruction, le au moins un paramètre de reconstruction étant un niveau keV de reconstruction, dans lequel le niveau keV de reconstruction a un impact à la fois sur les artefacts de balayage et sur le rapport contraste à bruit.

2. Le procédé mis en œuvre par ordinateur suivant la revendication 1,
dans lequel au moins l'une de la pluralité de fonctions de sous-coût est un terme de pénalité, le terme de pénalité étant, en particulier, pondéré par un paramètre de pondération.

3. Le procédé mis en œuvre par ordinateur suivant la revendication 2,
dans lequel au moins un terme de pénalité met en rapport au moins une de l'information se rapportant au balayage avec au moins l'un des paramètres d'imagerie.

4. Le procédé mis en œuvre par ordinateur suivant l'une quelconque des revendications précédentes,
dans lequel la fonction d'optimisation comprend une fonction de réaction à une dose, la fonction de réaction à une dose dépendant des au moins deux paramètres d'imagerie.

5. Le procédé mis en œuvre par ordinateur suivant l'une quelconque des revendications précédentes,
dans lequel l'information se rapportant au balayage comprend une dimension du sujet (5), en particulier une taille d'un patient.

6. Le procédé mis en œuvre par ordinateur suivant l'une quelconque des revendications précédentes,
dans lequel l'information se rapportant au balayage comprend un diamètre du sujet (5) en une région à balayer à laquelle on s'intéresse.

7. Le procédé mis en œuvre par ordinateur suivant l'une quelconque des revendications 5 ou 6 précédentes, comprenant les stades de
- réception d'un tomodensitogramme ou d'une image de caméra du sujet, dans lequel le tomodensitogramme ou l'image de caméra a été acquise, de préférence au début de l'examen de tomodensitométrie assisté par ordinateur, dans lequel le balayage de tomodensitométrie assisté par ordinateur, dont des paramètres d'imagerie sont adaptés, doit être acquis ;
- déduire la taille ou le diamètre du sujet du tomodensitogramme ou de l'image de caméra.

8. Le procédé mis en œuvre par ordinateur suivant l'une quelconque des revendications précédentes,
dans lequel les paramètres d'imagerie comprennent un réglage de filtre en coin.

9. Le procédé mis en œuvre par ordinateur suivant l'une quelconque des revendications précédentes,
dans lequel adapter des paramètres d'imagerie comprend, en fonction de l'information se rapportant au balayage, en particulier en fonction de la taille du sujet, déterminer un filtre en coin à utiliser parmi une pluralité d'au moins deux filtres en coin disponibles.

10. Le procédé mis en œuvre par ordinateur suivant l'une quelconque des revendications précédentes,
dans lequel adapter des paramètres d'imagerie comprend choisir un mode de balayage informatisé de tomodensitométrie parmi une pluralité de modes disponibles de balayage informatisé de tomodensitométrie.

11. Le procédé mis en œuvre par ordinateur suivant l'une quelconque des revendications précédentes,
dans lequel les paramètres d'imagerie sont des paramètres de balayage comprenant au moins l'un de ce qui suit : un réglage de filtre kV, un réglage de filtre spectral, un réglage de collimation, un facteur de pas spiral et un réglage de temps de rotation.

12. Un programme d'ordinateur comprenant des instructions, qui, lorsque le programme est exécuté par ordinateur, font que l'ordinateur effectue le procédé de l'une quelconque des revendications précédentes.

13. Un système (1) informatisé de tomodensitométrie comprenant une unité (2) de traitement, qui est configurée pour effectuer le procédé suivant l'une quelconque des revendications 1 à 11.
